# EUROPÄISCHE PATENTSCHRIFT

(11) **EP 2 085 100 B1**
(45) Veröffentlichungstag und Bekanntmachung des Hinweises auf die Patenterteilung: **28.01.2015**
(21) Anmeldenummer: 08171043.6
(22) Anmeldetag: 09.12.2008
(51) Int. Cl.: A61L 31/02, A61L 31/10, A61L 31/14

(54) **Implantat mit einem Grundkörper aus einer biokorrodierbaren Legierung und einer korrosionshemmenden Beschichtung**
Implant with a base body made of a biocorrodible alloy and a biocorrosion-reducing coating
Implant composé d'un alliage biocorrodable et d'un revêtement anticorrosion

(30) Priorität: 29.01.2008 DE 102008006455
(43) Veröffentlichungstag der Anmeldung: 05.08.2009
(73) Patentinhaber: Biotronik VI Patent AG, 6341 Baar (CH)
(72) Erfinder: Borck, Alexander, 91086, Aurachtal (DE)
(74) Vertreter: Lindner-Vogt, Karin L.

(56) Entgegenhaltungen:
- DE-A1- 19 843 254
- DE-A1-102005 018 356
- US-A1- 2002 122 877
- US-A1- 2005 112 170
- US-A1- 2006 246 108
- US-A1- 2007 014 848
- US-A1- 2007 224 244
- R K Chang ET AL: "Aliphatic polyesters" In: "Handbook of Pharmaceutical Excipients, Third Edition", 1 January 2000 (2000-01-01), American Pharmaceutical Association, Pharmaceutical Press, Washington, US, XP055071261, pages 14-15,
- ZHAO H ET AL: "Preparation of biodegradable magnetic microspheres with poly(lactic acid)-coated magnetite", JOURNAL OF MAGNETISM AND MAGNETIC MATERIALS, ELSEVIER SCIENCE PUBLISHERS, AMSTERDAM, NL, vol. 321, no. 10, 1 May 2009 (2009-05-01), pages 1356-1363, XP026052248, ISSN: 0304-8853, DOI: 10.1016/J.JMMM.2009.02.038 [retrieved on 2009-02-20]

## Beschreibung

Die Erfindung betrifft ein Implantat mit einem Grundkörper, der ganz oder in Teilen aus einem biokorrodierbaren metallischen Werkstoff besteht. Zumindest die aus dem biokorrodierbaren metallischen Werkstoff bestehenden Teile des Grundkörpers sind mit einer korrosionshemmenden Beschichtung bedeckt. Die Beschichtung umfasst eine Haftvermittlerschicht aus einem ersten biodegradierbaren polymeren Werkstoff und eine auf der Haftvermittlerschicht aufgebrachte Schutzschicht aus einem zweiten biodegradierbaren polymeren Werkstoff.

### Technologischer Hintergrund und Stand der Technik

Implantate finden in vielfältiger Ausführungsform in der modernen Medizintechnik Anwendung. Sie dienen unter anderem der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

Die Implantation von Stents ist eine der wirkungsvollsten therapeutischen Maßnahmen bei der Behandlung von Gefäßerkrankungen. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper jedes Implantats, insbesondere von Stents, besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Für die Zwecke der vorliegenden Erfindung sind lediglich degradierbare/resorbierbare, metallische Implantatwerkstoffe von Interesse, die nachfolgend als biokorrodierbare metallische Werkstoffe bezeichnet werden.

Der Einsatz biokorrodierbarer metallischer Werkstoffe bietet sich insbesondere schon deshalb an, da häufig nur ein zeitweiliger Verbleib des Implantats im Körper zur Erfüllung des medizinischen Zweckes erforderlich ist. Implantate aus permanenten Werkstoffen, also Werkstoffen, die im Körper nicht abgebaut werden, sind gegebenenfalls wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht demnach darin, das Implantat ganz oder in Teilen aus einem biokorrodierbaren metallischen Werkstoff zu formen. Unter Biokorrosion werden Vorgänge verstanden, die durch die Anwesenheit von Körpermedien bedingt sind und die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Die Abbauprodukte haben, wie beispielsweise beim Magnesium, im günstigsten Fall sogar eine positive therapeutische Wirkung auf das umgebende Gewebe. Geringe Mengen nicht resorbierbarer Legierungsbestandteile sind - sofern sie nicht toxische sind - tolerierbar.

Bekannte biokorrodierbare metallische Werkstoffe umfassen Reineisen und biokorrodierbare Legierungen der Hauptelemente Magnesium, Eisen, Zink, Molybdän und Wolfram. In DE 197 31 021 A1 wird unter anderem vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines Stent, eignet. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Insbesondere limitiert die relativ rasche Biokorrosion der Magnesiumlegierungen deren Einsatzmöglichkeiten.

Herkömmliche technische Einsatzgebiete von Formkörpern aus metallischen Werkstoffen, insbesondere aus Magnesiumlegierungen, außerhalb der Medizintechnik erfordern in der Regel eine weitgehende Unterbindung korrosiver Prozesse. Dementsprechend ist die Zielstellung der meisten technischen Verfahren zur Verbesserung des Korrosionsverhaltens eine vollständige Inhibierung korrosiver Prozesse. Dagegen sollte die Zielstellung zur Verbesserung des Korrosionsverhaltens der hier vorliegenden biokorrodierbaren metallischen Werkstoffe nicht in der vollständigen Unterbindung, sondern nur in einer temporären Hemmung der korrosiven Prozesse liegen. Schon aus diesem Grunde eignen sich die meisten bekannten Maßnahmen zur Verbesserung des Korrosionsschutzes nicht. Ferner müssen für einen medizintechnischen Einsatz auch toxikologische Aspekte berücksichtigt werden. Weiterhin sind korrosive Prozesse stark von dem Medium abhängig, in dem sie ablaufen; eine Übertragung der unter herkömmlichen Umweltbedingungen auf technischem Gebiet gewonnenen Erkenntnisse über die Eigenschaften spezifischer Antikorrosionsschutzbeschichtungen auf die Prozesse in physiologischer Umgebung ist daher in der Regel nicht möglich.

Ein Ansatzpunkt bekannter technischer Verfahren zur Verbesserung des Korrosionsverhaltens (im Sinne einer Verstärkung des Korrosionsschutzes) sieht vor, eine korrosionsschützende Schicht auf dem aus dem metallischen Werkstoff bestehenden Formkörper zu erzeugen. Bekannte Verfahren zur Erzeugung einer korrosionsschützenden Schicht wurden unter dem Gesichtspunkt eines technischen Einsatzes des beschichteten Formkörpers - jedoch nicht medizintechnischen Einsatzes in biokorrodierbaren Implantaten in physiologischer Umgebung - entwickelt und optimiert. Diese bekannten Verfahren umfassen beispielsweise: das Aufbringen von Polymeren oder anorganischen Deckschichten, das Erzeugen einer Emaille, die chemische Konversion der Oberfläche, Heißgasoxidation, Anodisieren, Plasmaspritzen, Laserstrahl-Umschmelzen, PVD-Verfahren, Ionenimplantation oder Lackieren.

EP 1 389 471 B1 beschreibt einen Stent mit einem Grundkörper aus einem biokorrodierbaren metallischen Werkstoff, insbesondere einer Magnesiumlegierung. Die Implantatsoberfläche trägt eine polymere Beschichtung aus einem hochmolekularen Poly(L-lactid). Eine Haftvermittlerschicht kann zwischen der Implantatsoberfläche und der polymeren Beschichtung vorgesehen sein.

DE 198 43 254 A1 beschreibt Implantate mit einer Beschichtung aus einem Polymergemisch, das Cyanacrylat oder Methylenmalonester enthält. Das Polymergemisch kann Poly(D,L-lactid-co-glycolide) enthalten.

US2005/0112170 beschreibt eine biokorrodierbare medizinische Vorrichtung, welche eine Beschichtung aus einem biodegradierbaren Polymer aufweist, wobei das Polymer ein Poly(D,L-lactid) oder ein Diblockcopolymer bestehen aus Poly(D,L-lactid) mit Polyethylenglycol PEG sein kann.

US2007/0014848 offenbart ein biokorrodierbares medizinisches Implantat, welches ein Blockcopolymer bestehend aus einer Polyestereinheit und einer Polyethereinheit PEG aufweist.

Der vorliegenden Erfindung liegt die Aufgabe zugrunde, eine verbesserte oder zumindest alternative Beschichtung für ein Implantat aus einem biokorrodierbaren metallischen Werkstoff bereitzustellen, die eine temporäre Inhibition, aber nicht vollständige Unterbindung der Korrosion des Werkstoffs in physiologischer Umgebung bewirkt.

### Zusammenfassung der Erfindung

Die Erfindung geht aus von einem Implantat mit einem Grundkörper, der ganz oder in Teilen aus einem biokorrodierbaren metallischen Werkstoff besteht. Zumindest die aus dem biokorrodierbaren metallischen Werkstoff bestehenden Teile des Grundkörpers sind mit einer korrosionshemmenden Beschichtung bedeckt. Die Beschichtung umfasst eine Haftvermittlerschicht aus einem ersten biodegradierbaren polymeren Werkstoff und eine auf der Haftvermittlerschicht aufgebrachte Schutzschicht aus einem zweiten biodegradierbaren polymeren Werkstoff. Das Implantat zeichnet sich dadurch aus, dass
(i) der erste biodegradierbare polymere Werkstoff der Haftvermittlerschicht ein Poly(D,L-lactid) (PDLLA) mit einem Polymerisationsgrad im Bereich von 5 bis 20 ist; und
(ii) der zweite biodegradierbare polymere Werkstoff der Schutzschicht ein Diblockcopolymer (PEG/PLGA) aus Polyethylenglycol (PEG) und Poly(D,L-lactid-co-glycolide) (PLGA) ist.

Es hat sich gezeigt, dass die Aufbringung einer Beschichtung der genannten Zusammensetzung nicht zur Ausbildung einer permanent vollständig oder weitgehend die Korrosion in physiologischer Umgebung inhibierenden Schutzschicht führt. Mit anderen Worten, in physiologischer Umgebung erfolgt dennoch eine Korrosion des Implantats, jedoch mit deutlich verzögerter Geschwindigkeit. Vorteilhaft ist insbesondere auch, dass nur Deckschichten mit sehr geringer Schichtdicke des Polymers benötigt werden. Das hat den Vorteil, dass eine Adaption bewährter Geometrien der Implantatgrundkörper zumeist entfällt, also zum Beispiel bei Stent auch Streben mit sehr geringem Durchmesser beschichtet werden können. Damit einhergehend sind die Mengen an Polymer auf dem Implantat verringert, so dass schon aus diesem Grunde gegenüber herkömmlichen Beschichtungen unerwünschte Gewebsreaktionen vermindert werden.

Vorzugsweise ist der biokorrodierbare metallische Werkstoff eine biokorrodierbare Legierung ausgewählt aus der Gruppe der Elemente Magnesium, Eisen, Zink, Molybdän und Wolfram; insbesondere ist der Werkstoff eine biokorrodierbare Magnesiumlegierung. Unter Legierung wird vorliegend ein metallisches Gefüge verstanden, deren Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Diese Magnesiumlegierung bestätigte bereits in klinischen Versuchen ihre besondere Eignung, d. h. zeigt eine hohe Biokompatibilität, günstige Verarbeitungseigenschaften, gute mechanische Kennwerte. Durch in vivo Studien konnte gezeigt werden, dass die Magnesiumlegierung abgebaut beziehungsweise durch körpereigene Bestandteile ersetzt wird. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Bevorzugt sind weiterhin Magnesiumlegierungen die bis zu 6 Gew.% Zink enthalten. Besonders bevorzugt ist weiterhin eine Magnesiumlegierung der Zusammensetzung Yttrium 0,5 - 10 Gew.%, Zink 0,5 - 6 Gew.%, Calcium 0,05 - 1 Gew.%, Mangan 0 - 0,5 Gew.%, Silber 0 - 1 Gew.%, Cer 0 - 1 Gew.% sowie Zirkonium 0 - 1 Gew.% oder Silizium 0 - 0,4 Gew.%, wobei sich die Angaben auf Gew.% an der Legierung beziehen und Magnesium sowie herstellungsbedingte Verunreinigungen den auf 100 Gew.% verbleibenden Restanteil an der Legierung einnehmen.

Die Legierungen der Elemente Magnesium, Eisen, Zink, Molybdän oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaC1 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Im Fall von Koronarstents sollte vorzugsweise die mechanische Integrität der Struktur über ein Zeitraum von drei Monaten oder mehr nach Implantation aufrechterhalten werden.

Implantate im Sinne der Erfindung sind über ein chirurgisches oder minimalinvasives Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes, beispielsweise Stents, und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtung ganz oder zumindest weitgehend diesen Anforderungen genügt.

Poly(D,L-lactid) (PDLLA) weist eine terminale Carbonsäurefunktion auf, von der bekannt ist, dass sie an Magnesiumoberflächen Carboxylate bilden kann. Die Anbindung über Carboxylate gewährt die für die Einsatzzwecke notwendige Bindungskraft der Haftvermittlerschicht auf der Implantatsoberfläche. Vorzugsweise weist das Poly(D,L-lactid) (PDLLA) der Haftvermittlerschicht einen Polymerisationsgrad von 12 auf.

Bevorzugt ist weiterhin, wenn das Diblockcopolymer (PEG/PLGA) Polymerblöcke aus Polyethylenglycol (PEG) mit einem Molekulargewicht im Bereich von 2.000 bis 8.000 g/mol enthält.

Die Polymerblöcke aus Polyethylenglycol (PEG) tragen vorzugsweise 1 bis 20 % zum Gesamtgewicht des Diblockcopolymers (PEG/PLGA) bei.

Ferner ist bevorzugt, wenn das Diblockcopolymer (PEG/PLGA) eine inhärente Viskosität im Bereich von 0,5 bis 2 dl/g (0,1 %, CHCl₃, 25 °C) besitzt. Besonders bevorzugt liegt die inhärente Viskosität bei 1 dl/g.

Nach einer weiteren, bevorzugten Ausführungsform weisen die Polymerblöcke aus Poly(D,L-lactid-co-glycolide) (PLGA) Monomerenverhältnisse zwischen 2 : 1 und 1 : 2, insbesondere ein Monomerenverhältnis von 1 : 1 auf.

Schließlich ist nach einer bevorzugten Ausführungsform das Implantat ein Stent und die aufgebrachte Menge des Diblockcopolymers (PEG/PLGA) beträgt 15 bis 20 µg pro mm Stentlänge. Bei Schichtmassen unterhalb der angegebenen Untergrenze ist eine homogene Bedeckung der zu beschichtenden Bereiche des Grundkörpers nicht mehr sichergestellt, so dass eine reproduzierbare Einstellung eines gewünschten Korrosionsverhaltens erschwert ist. Oberhalb der genannten Grenze für die Schichtmasse können Eigenspannungen innerhalb der Schicht auftreten, die zu Inhomogenitäten führen, was wiederum eine reproduzierbare Einstellung des gewünschten Korrosionsverhaltens erschweren kann. Es versteht sich, dass mit steigender Schichtmasse die korrosionshemmende Wirkung der Beschichtung zunimmt. Zur Erzielung eines vorgebbaren Korrosionsverhaltens kann der Fachmann wie folgt vorgehen:

Es werden Probenkörper aus dem biokorrodierbaren metallischen Werkstoff hergestellt und diese werden mit einer Haftvermittlerschicht und dann Schutzschicht einer jeweils vorgebbaren Schichtmasse bedeckt. Auf diese Weise können beispielsweise fünf Probenkörper mit unterschiedlichen Schichtmassen hergestellt werden, deren Korrosionsverhalten anschließend quantifiziert wird (zum Beispiel durch Bestimmung der Korrosionsrate) und die eine qualitative Voraussage des Zusammenhangs zwischen Schichtmasse und Korrosionsverhalten erlauben. Die erhaltenen Daten für das Korrosionsverhalten werden mit dem gewünschten Korrosionsverhalten verglichen. Zeigt der Vergleich noch signifikante Abweichungen zu jedem der von den Probenkörpern erhaltenen Werte, wird ausgehend von dem am nächsten liegenden Wert die Schichtmasse in weiteren Probenkörpern variiert. Letztendlich kann der Fachmann durch routinemäßiges Abarbeiten dieser Optimierungsprozedur eine Schichtmasse für das gewünschte Korrosionsverhalten ermitteln.

### Kurzbeschreibung der Zeichnungen

Die Erfindung wird nachfolgend anhand eines Ausführungsbeispiels und den dazugehörigen Zeichnungen näher erläutert. Es zeigen:
- Figur 1: Das Abbauverhalten eines erfindungsgemäß modifizierten Stents im Vergleich zu einem unbeschichteten Stent, einem nur mit einer Haftvermittlerschicht bedeckten Stent und einem mit einer Haftvermittlerschicht und einer nicht erfindungsgemäßen Schutzschicht bedeckten Stent; und
- Figur 2: Das Abbauverhalten eines erfindungsgemäß modifizierten Stents in Abhängigkeit von der aufgebrachten Schichtmasse der Schutzschicht.

### Ausführungsbeispiele - Allgemeine Vorschriften

In den nachfolgenden Ausführungsbeispielen wurden Stents aus der kommerziell erwerbbaren Magnesiumlegierung WE43 (nach ASTM) mit einem Seltenerdmetallanteil von etwa 3 Gew.% ohne Yttrium und einem Yttriumanteil von etwa 4 Gew.% sowie einer Stentlänge von 10 mm beschichtet.

### Herstellung der Haftvermittlerschicht

Nach einer ersten Variante wurde eine Suspension von 200 mg Poly(D,L-lactid) mit einem Polymerisationsgrad von 12 in 10 ml absolutem Ethanol angesetzt. In diese Suspension wurde der Stent für 60h bei Raumtemperatur inkubiert, dann entnommen, mit absolutem Ethanol gespült und an der Luft getrocknet.

Nach einer zweiten Variante wurde eine Lösung von 200 mg Poly(D,L-lactid) mit einem Polymerisationsgrad von 12 in 10 ml absolutem Chloroform angesetzt. In diese Lösung wurde der Stent für 60h bei Raumtemperatur inkubiert, dann entnommen und an der Luft getrocknet.

### Herstellung der Schutzschicht

Es wurde eine 0,1 Gew.% Lösung eines Diblockcopolymer (PEG/PLGA) aus Polyethylenglycol (PEG) und Poly(D,L-lactid-co-glycolide) (PLGA) in absolutem Aceton angesetzt, bei dem das Diblockcopolymer (PEG/PLGA) Polymerblöcke aus Polyethylenglycol (PEG) mit einem Molekulargewicht im Bereich von 5.000 g/mol enthält, die Polymerblöcke aus Polyethylenglycol (PEG) 5 % zum Gesamtgewicht des Diblockcopolymers (PEG/PLGA) beitragen sowie die Polymerblöcke aus Poly(D,L-lactid-co-glycolide) (PLGA) ein Monomerenverhältnis von 1 : 1 aufweisen (erhältlich unter dem Handelnamen Resomer RGP d 5055 bei der Firma Boehringer Ingelheim). Die Lösung wurde auf einen zu beschichtenden Stents gesprüht und anschließend wurde an der Luft getrocknet.

### Ausführungsbeispiel 1 - Stent mit Haftvermittlerschicht und Schutzschicht

Ein nach dem obig beschriebenen Verfahren hergestellter Stent mit einer Haftvermittlerschicht wurde entsprechend dem obig beschriebenen Verfahren mit einer Schutzschicht beschichtet. Der Sprühvorgang wurde so oft wiederholt, bis die Masse der aufgetragenen Schutzschicht 75 µg betrug.

### Ausführungsbeispiel 2

Ein Stent mit einer Haftvermittlerschicht und Schutzschicht wurde in gleicher weise, wie im Ausführungsbeispiel 1 beschrieben, hergestellt. Der Sprühvorgang wurde jedoch so oft wiederholt, bis die Masse der aufgetragenen Schutzschicht 200 µg betrug.

### Ausführungsbeispiel 3

Ein Stent mit einer Haftvermittlerschicht und Schutzschicht wurde in gleicher weise, wie im Ausführungsbeispiel 1 beschrieben, hergestellt. Der Sprühvorgang wurde jedoch so oft wiederholt, bis die Masse der aufgetragenen Schutzschicht 350 µg betrug.

### Vergleichsbeispiel 1 - Stent ohne Beschichtung

Zu Vergleichszwecken wurde ein Stent ohne Beschichtung verwendet.

### Vergleichsbeispiel 2 - Stent mit Haftvermittlerschicht

Zu Vergleichszwecken wurde ein Stent mit einer entsprechend der oben beschriebenen zweiten Verfahrensvariante erzeugten Haftvermittlerschicht hergestellt.

### Vergleichsbeispiel 3 - Stent mit Haftvermittlerschicht und nicht erfindungsgemäßer biokorrodierbarer Schutzschicht

Zu Vergleichszwecken wurde ein Stent mit einer entsprechend dem oben beschriebenen Verfahren erzeugten Haftvermittlerschicht und einer zur Erfindung alternativen Schutzschicht hergestellt. Diese Schutzschicht besteht aus einem niedermolekularem Poly(D,L-Lactid-co-glycolid) PLGA 85:15, mit einer inhärenten Viskosität von 0,63 dl/g und wurde analog zur Herstellung der erfindungsgemäßen Schutzschicht aufgetragen.

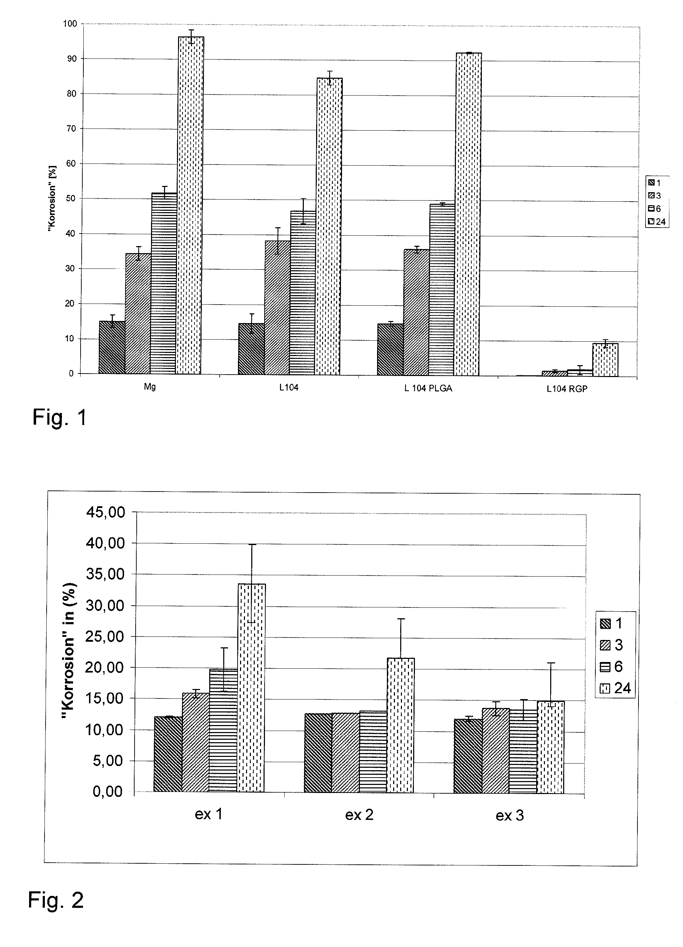

Figur 1 illustriert das Ausmaß der Korrosion der Stents gemäß dem Ausführungsbeispiel 1 (in der Figur bezeichnet als L104 RGP) und der Vergleichsbeispiele 1 bis 3 (in der Figur bezeichnet als Mg / L104 / L104 PLGA). Dazu wurden die Stents in einer PBS-Lösung (phosphat-gepufferte Salzlösung der Zusammensetzung: 8 g NaCl / 0.2 g KCI / 1.44 g Na₂HPO₄ / 0.24 g KH₂PO₄ in 1 I H₂O, eingestellt auf pH 7.4) für 1, 3, 6 und 24 h inkubiert. Wie ersichtlich, führt eine Kombination aus Haftvermittlerschicht und erfindungsgemäßer Schutzschicht zu einer erheblichen Hemmung der Korrosion des Stents, jedoch nicht zu einer vollständigen Unterbindung des Prozesses. Das Ausmaß der Korrosion ist dabei dergestalt, dass die Stützkraft des Stents auch nach 24h noch weitgehend erhalten ist.

Figur 2 zeigt den Einfluss der Schichtmasse auf das Ausmaß der Korrosion in einer PBS-Lösung nach 1, 3, 6 und 24 h. Es werden die Stents der Ausführungsbeispiele 1 bis 3 gegenüber gestellt (in der Figur bezeichnet als ex1 / ex2 / ex3). Wie ersichtlich, lässt sich das Korrosionsverhalten durch Variation der aufgetragenen Masse an Material für die Schutzschicht beeinflussen. Je mehr Masse aufgetragen wird, desto länger wird die Korrosion verzögert.

## Patentansprüche

1. Implantat mit einem Grundkörper, der ganz oder in Teilen aus einem biokorrodierbaren metallischen Werkstoff besteht und bei dem zumindest die aus dem biokorrodierbaren metallischen Werkstoff bestehenden Teile des Grundkörpers mit einer korrosionshemmenden Beschichtung bedeckt sind, die eine Haftvermittlerschicht aus einem ersten biodegradierbaren polymeren Werkstoff und eine auf der Haftvermittlerschicht aufgebrachte Schutzschicht aus einem zweiten biodegradierbaren polymeren Werkstoff umfasst, **dadurch gekennzeichnet, dass**
(i) der erste biodegradierbare polymere Werkstoff der Haftvermittlerschicht ein Poly(D,L-lactid) (PDLLA) mit einem Polymerisationsgrad im Bereich von 5 bis 20 ist; und
(ii) der zweite biodegradierbare polymere Werkstoff der Schutzschicht ein Diblockcopolymer (PEG/PLGA) aus Polyethylenglycol (PEG) und Poly(D,L-lactid-co-glycolide) (PLGA) ist.

2. Implantat nach Anspruch 1, bei dem der biokorrodierbare metallische Werkstoff eine biokorrodierbare Legierung ausgewählt aus der Gruppe Magnesium, Eisen, Zink, Molybdän und Wolfram ist.

3. Implantat nach Anspruch 2, bei dem der biokorrodierbare metallische Werkstoff eine Magnesiumlegierung ist.

4. Implantat nach einem der vorhergehenden Ansprüche, bei dem das Implantat ein Stent ist.

5. Implantat nach einem der vorhergehenden Ansprüche, bei dem das Poly(D,L-lactid) (PDLLA) der Haftvermittlerschicht einen Polymerisationsgrad von 12 aufweist.

6. Implantat nach einem der vorhergehenden Ansprüche, bei dem das Diblockcopolymer (PEG/PLGA) Polymerblöcke aus Polyethylenglycol (PEG) mit einem Molekulargewicht im Bereich von 2.000 bis 8.000 g/mol enthält.

7. Implantat nach einem der vorhergehenden Ansprüche, bei dem die Polymerblöcke aus Polyethylenglycol (PEG) 1 bis 20 % zum Gesamtgewicht des Diblockcopolymers (PEG/ PLGA) beitragen.

8. Implantat nach einem der vorhergehenden Ansprüche, bei dem das Diblockcopolymer (PEG/PLGA) eine inhärente Viskosität im Bereich von 0.5 bis 2 dl/g, 0,1% in Chloroform bei 25 °C, besitzt.

9. Implantat nach einem der vorhergehenden Ansprüche, bei dem die Polymerblöcke aus Poly(D,L-lactid-co-glycolide) (PLGA) Monomerenverhältnisse zwischen 2 : 1 und 1 : 2 aufweisen.

10. Implantat nach Anspruch 9, bei dem die Polymerblöcke aus Poly(D,L-lactid-co-glycolide) (PLGA) ein Monomerenverhältnis von 1 : 1 aufweisen.

11. Implantat nach Anspruch 4, bei dem die aufgebrachte Menge des Diblockcopolymers (PEG/PLGA) 15 bis 20 pg pro mm Stentlänge beträgt.

## Claims

1. An implant comprising a base body, which is made entirely or in parts of a biocorrodible metallic material and in which, at the least, the parts of the base body that are made of the biocorrodible metallic material are covered with a corrosion-inhibiting coating, which comprises a primer layer made of a first biodegradable polymeric material and a protective layer, which is made of a second biodegradable polymeric material and is applied on the primer layer, **characterized in that**
(i) the first biodegradable polymeric material of the primer layer is a poly(D, L-lactide) (PDLLA) having a degree of polymerization in the range from 5 to 20; and
(ii) the second biodegradable polymeric material of the protective layer is a diblock copolymer (PEG/PLGA) comprising polyethylene glycol (PEG) and poly(D, L-lactide-co-glycolide) (PLGA).

2. The implant according to claim 1, wherein the biocorrodible metallic material is a biocorrodible alloy selected from the group of magnesium, iron, zinc, molybdenum, and tungsten.

3. The implant according to claim 2, wherein the biocorrodible metallic material is a magnesium alloy.

4. The implant according to one of the preceding claims, wherein the implant is a stent.

5. The implant according to any one of the preceding claims, wherein the poly(D, L-lactide) (PDLLA) of the primer layer has a degree of polymerization of 12.

6. The implant according to any one of the preceding claims, wherein the diblock copolymer (PEG/PLGA) contains polymer blocks comprising polyethylene glycol (PEG) having a molecular weight in the range from 2,000 to 8,000 g/mol.

7. The implant according to any one of the preceding claims, wherein the polymer blocks comprising polyethylene glycol (PEG) contribute 1 to 20% to the overall weight of the diblock copolymer (PEG/PLGA).

8. The implant according to any one of the preceding claims, wherein the diblock copolymer (PEG/PLGA) has an inherent viscosity in the range from 0.5 to 2 dl/g, 0.1% in chloroform at 25°C.

9. The implant according to any one of the preceding claims, wherein the polymer blocks comprising poly(D, L-lactide-co-glycolide) (PLGA) have monomer ratios between 2 : 1 and 1 : 2.

10. The implant according to claim 9, wherein the polymer blocks comprising poly(D, L-lactide-co-glycolide) (PLGA) have a monomer ratio of 1:1.

11. The implant according to claim 4, wherein the amount of the diblock copolymer (PEG/PLGA) that is applied is 15 to 20 pg per mm stent length.

## Revendications

1. Implant avec un corps de base constitué entièrement ou partiellement d'un matériau métallique bio-corrodable et dans lequel au moins les parties du corps de base qui sont constituées du matériau métallique bio-corrodable sont recouvertes d'un revêtement anticorrosion, comprenant une couche de promoteur d'adhérence constituée d'un premier matériau polymère biodégradable et une couche de protection constituée d'un deuxième matériau polymère biodégradable, appliquée sur la couche de promoteur d'adhérence, **caractérisé en ce que**
(i) le premier matériau polymère biodégradable de la couche de promoteur d'adhérence est un poly(D,L-lactide) (PDLLA) avec un degré de polymérisation dans la plage de 5 à 20 ; et
(ii) le deuxième matériau polymère biodégradable de la couche de protection est un polymère di-bloc (PEG/PLGA) constitué de polyéthylène glycol (PEG) et de poly(D,L-lactide-co-glycolide) (PLGA).

2. Implant selon la revendication 1, dans lequel le matériau métallique bio-corrodable est un alliage bio-corrodable sélectionné parmi le groupe comprenant le magnésium, le fer, le zinc, le molybdène et le wolfram.

3. Implant selon la revendication 2, dans lequel le matériau métallique bio-corrodable est un alliage de magnésium.

4. Implant selon l'une des revendications précédentes, dans lequel l'implant est un stent.

5. Implant selon l'une des revendications précédentes, dans lequel le poly(D,L-lactide) (PDLLA) de la couche de promoteur d'adhérence présente un degré de polymérisation de 12.

6. Implant selon l'une des revendications précédentes, dans lequel le copolymère di-bloc (PEG/PLGA) contient des blocs de polymères constitués de polyéthylène glycol (PEG) avec un poids moléculaire dans la plage de 2000 à 8000 g/mol.

7. Implant selon l'une des revendications précédentes, dans lequel les blocs de polymères constitués de polyéthylène glycol (PEG) contribuent à raison de 1 à 20% au poids global du copolymère di-bloc (PEG/PLGA).

8. Implant selon l'une des revendications précédentes, dans lequel le copolymère di-bloc (PEG/PLGA) présente une viscosité inhérente dans la plage de 0,5 à 2 dl/g, 0,1% dans du chloroforme à 25°C.

9. Implant selon l'une des revendications précédentes, dans lequel les blocs de polymères constitués de poly(D,L-lactide-co-glycolide) (PLGA) présentent des rapports de monomères entre 2:1 et 1:2.

10. Implant selon la revendication 9, dans lequel les blocs de polymères constitués de poly(D,L-lactide-co-glycolide) (PLGA) présentent un rapport de monomères de 1:1.

11. Implant selon la revendication 4, dans lequel la quantité appliquée de copolymère di-bloc (PEG/PLGA) est comprise entre 15 et 10 pg par mm de stent.
